# EUROPEAN PATENT SPECIFICATION

(11) **EP 0 338 785 B1**
(45) Date of publication and mention of the grant of the patent: **07.07.1999**
(21) Application number: 89303849.7
(22) Date of filing: 19.04.1989
(51) Int. Cl.: G03C 7/26, C07C 243/24

(54) **Photographic element containing scavenger for oxidized developing agent**
Photographisches Element, das einen Fänger für das Entwickleroxidationsprodukt enthält
Elément photographique contenant un composé capable de bloquer un agent développateur oxydé

(30) Priority: 21.04.1988 US 184526
(43) Date of publication of application: 25.10.1989
(73) Proprietor: EASTMAN KODAK COMPANY (a New Jersey corporation), Rochester, New York 14650 (US)
(72) Inventor: Harder, John William c/o EASTMAN KODAK COMPANY, Rochester New York 14650 (US)
(74) Representative: Nunney, Ronald Frederick Adolphe

(56) References cited:
- EP-A- 0 242 685
- DE-C- 894 810
- US-A- 2 309 492
- US-A- 3 386 831

## Description

This invention relates to a photographic element, and in particular to a color photographic element containing a scavenger compound for oxidized developing agent.

It is known in the art to add a scavenger to a photographic element in order for the scavenger to interact with oxidized developing agent to prevent it from reacting within the element at an undesired location or at an undesired point in time. The presence of oxidized developing agent at an unwanted location can cause stain or fog and thereby affect the quality of the color images obtained.

Known scavengers for oxidized developing agents include ballasted hydroquinone compounds as described in U.S. Patent 3,700,453 wherein the ballasting groups are secondary alkyl substituents having from 9 to 20 carbon atoms. Also known are ballasted sulfonamidophenols such as described in U. S. Patent No. 4,205,987 and in Research Disclosure, February 1979, Item No. 17842. (Research Disclosure is published by Kenneth Mason Publications, Ltd., The Old Harbourmaster's, 8 North Street, Emsworth, Hampshire P010 7DD, ENGLAND, and is referred to hereinafter simply as "RD"). However, none of these references suggests use of hydrazide type compounds as scavenging agents for oxidized developer moieties.

U. S. Patent 4,684,604 relates to compounds capable of releasing photographically useful groups (PUG). The compounds comprise a hydrazide moiety which is capable of being oxidized to an azo group. The azo group then causes release of the desired PUG. The hydrazide moiety is attached to a heteroatom of a moiety containing the PUG, the attachment being through a link which comprises an acidic group or an active methylene group positioned adjacent to an acidic group. This patent does not disclose or suggest the use of hydrazide compounds for scavenging oxidized developer moieties.

U.S. Patent 4,224,401 discloses a process for producing high contrast negative silver images utilizing high sensitivity silver bromide or silver bromoiodide emulsions and hydrazide compounds. The hydrazide compounds function as nucleating agents and can comprise substituted phenyl groups. However, this U.S. Patent neither discloses nor suggests the use of such hydrazide compounds as scavengers for oxidized developing agents in color photographic elements.

Accordingly, it is desirable to reduce, or to eliminate, oxidized developing agent remaining in color photographic elements. Prior art hydrazine compounds have not been used or suggested for this purpose.

The object of the present invention is to provide a color photographic element which comprises a support, at least one silver halide emulsion layer and a scavenger compound for oxidized developing agent.

This object is achieved with a color photographic element which is characterized in that the scavenger compound is a hydrazide having the structural formula: 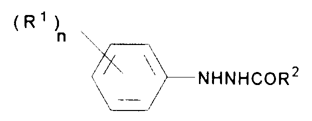 wherein:
R¹ is alkyl of 1 to 20 carbon atoms, alkoxy of 1 to 20 carbon atoms, carboxy, carbonamido having the formula -NR⁴COR⁵, -OCOR⁵, sulphonamido having the formula -NR⁴SO₂R⁵, or amino having the formula -NR⁴R⁵, wherein R⁴ is hydrogen or alkyl of 1 to 8 carbon atoms, and R⁵ is hydrogen, alkyl of 1 to 16 carbon atoms, a benzyl group or a phenyl group;
R² represents hydrogen, alkyl of 1 to 20 carbon atoms, alkoxy of 1 to 20 carbon atoms, aryl having 6 to 10 ring carbon atoms, aryloxy having 6 to 10 ring carbons, aralkyl or amino of the formula -NHR³ wherein R³ is phenyl or benzyl, and
n is 0, 1 or 2,
provided that at least one of the substituents R¹ and R² represents a ballast group of sufficient size as to render the hydrazide compound non-diffusible in the photographic element prior to development in alkaline processing solution,
further provided that at least one of the substituents R¹ and R² comprises a polar group which is -NHSO₂CH₃, -NHSO₂C₁₆H₃₃, -NHSO₂aryl, -CH₂OH, -NH₂, -COOH, -CONH₂, -NHCONH₂, -NHCSNH₂, -N⁺(R⁵)₃, -SO₃⁻, -SO₂⁻, 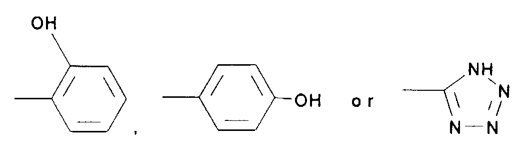 and still further provided that the hydrazide compound does not contain a timing group which releases a photographically useful group.

R¹ substituents, which are electron donating groups, include alkyl, which can be substituted or unsubstituted, straight or branched chain, having from 1 to 20 carbon atoms, preferably from 8 to 16 carbon atoms; alkoxy, which can be substituted or unsubstituted, straight or branched chain, having from 1 to 20 carbon atoms, preferably from 8 to 16 carbon atoms; and amino having the formula -NR⁴R⁵ where R⁴ is hydrogen or alkyl having from 1 to 8 carbon atoms and R⁵ is hydrogen, alkyl of 1 to 16 carbon atoms or is a benzyl or a phenyl group which may be substituted.
R² substitutents which are alkyl or alkoxy can be as defined for these same substitutents in R¹ or R² can be aryl or aryloxy having from 6 to 10 ring carbon atoms, such as phenyl, phenoxy, naphthyl or naphthoxy.

When R² represents phenyl or phenoxy it is preferred that the aryl ring have a hydrogen bonding substituent in a position ortho to the point of attachment of the carbonyl group to a hydrazide nitrogen atom. Preferred hydrogen bonding groups include hydroxy, primary or secondary amino groups of the formula -NR⁴R⁵, sulfonamido of the formula -NHSO₂R⁴, carbonamido of the formula -NR⁴COR⁵ and ureido of the formula -NHCONHR⁴ where R⁴ is hydrogen or alkyl of from 1 to 8 carbon atoms and R⁵ is hydrogen, alkyl of 1 to 16 carbon atoms, or a benzyl or phenyl group.

These groups can also be present as substituents on R² alkyl groups.

At least one of R¹ and R² comprises a polar group which is -NHSO₂CH₃, -NHSO₂C₁₆H₃₃, -NHSO₂aryl, -CH₂OH, -NH₂, -COOH, -CONH₂, -NHCONH₂, -NHCSNH₂, -N⁺(R⁵)₃, -SO₃⁻, -SO₂⁻,

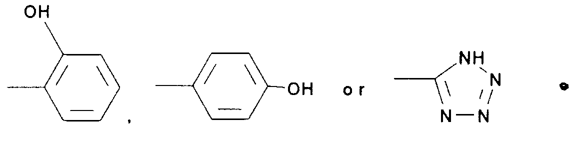

These groups tend to increase the surfactant nature of the hydrazine during alkaline processing.

As used herein and throughout the specification , reference to the alkyl, alkoxy, aryl, aryloxy, aralkyl and benzyl groups which are represented by one or more of R¹, R², R³, R⁴ and R⁵ includes substitution with halogen atoms, for example chlorine, or with haloalkyl groups, for example trifluoromethyl, or with 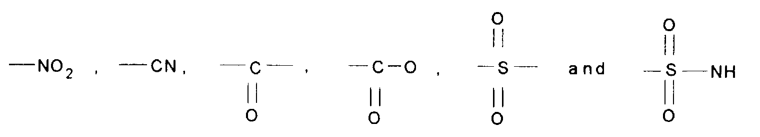 Typical compounds which fall within the above-presented structural formula include:

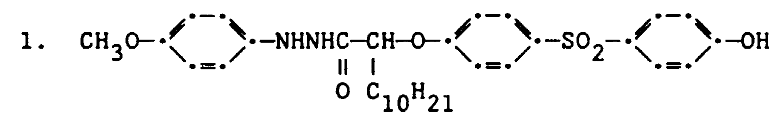

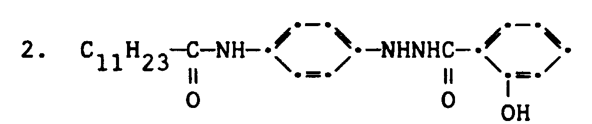

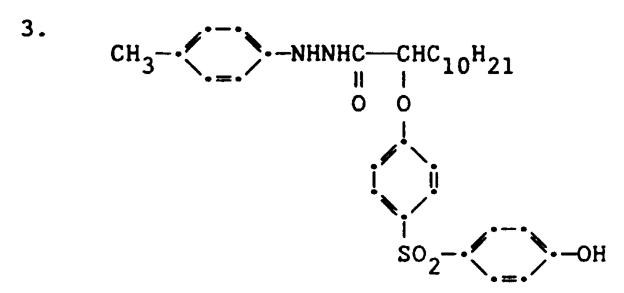

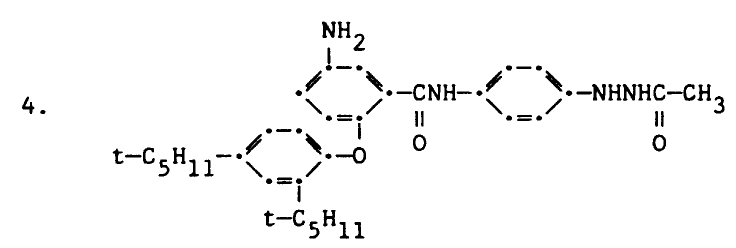

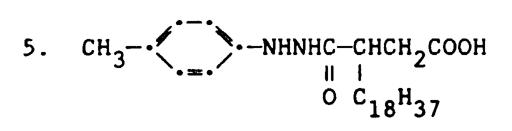

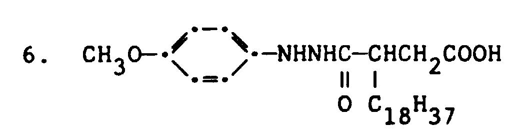

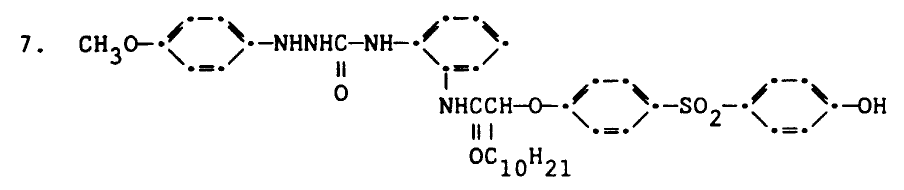

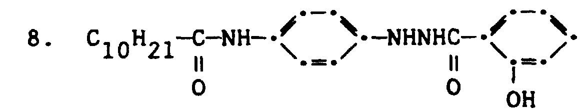

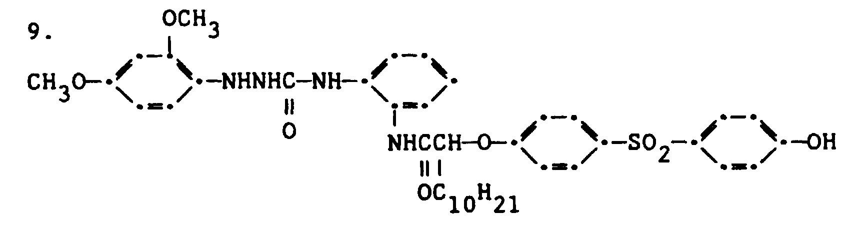

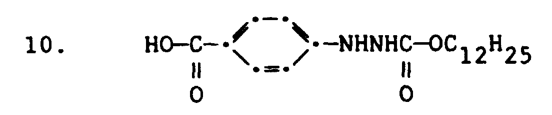

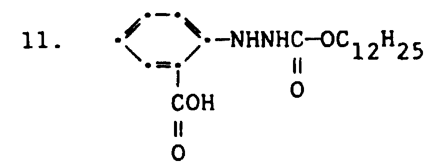

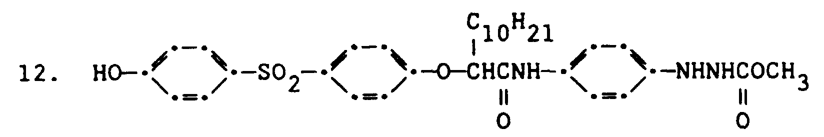

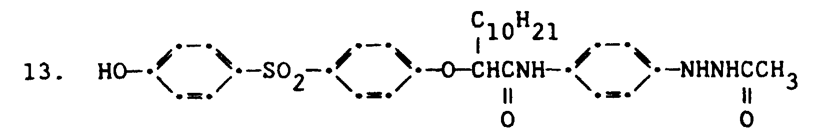

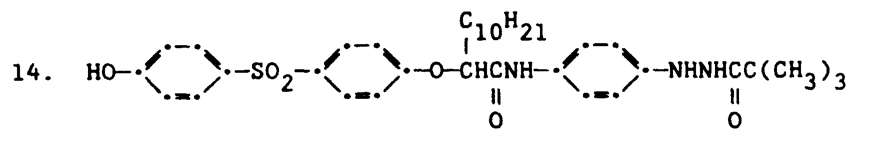

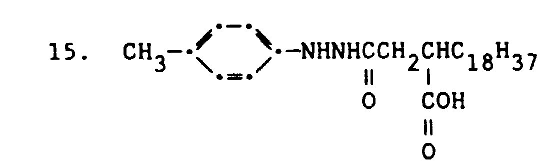

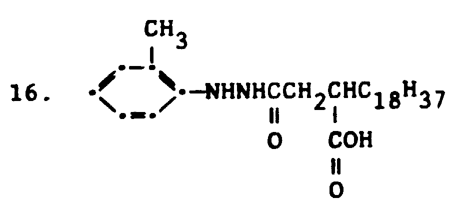

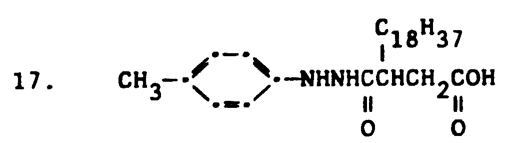

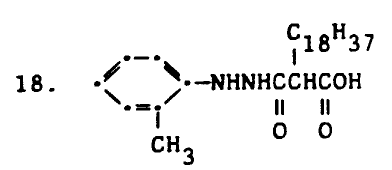

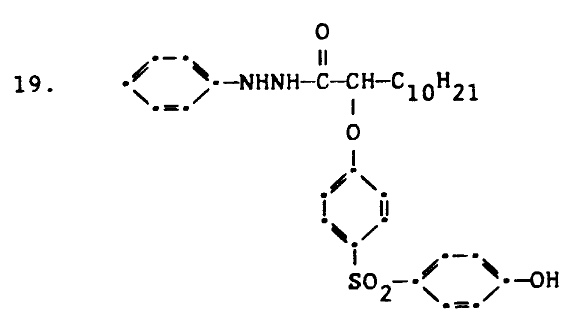

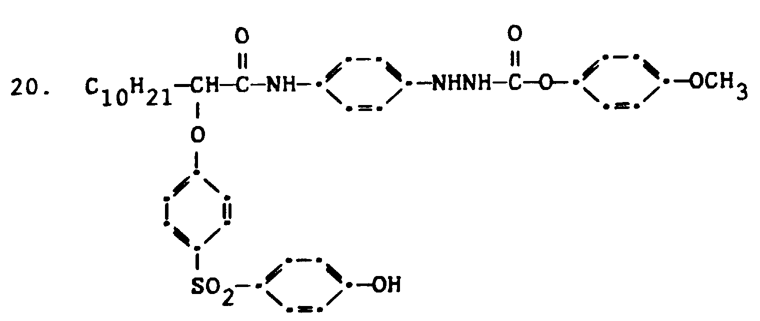

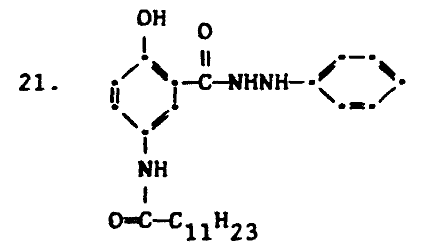

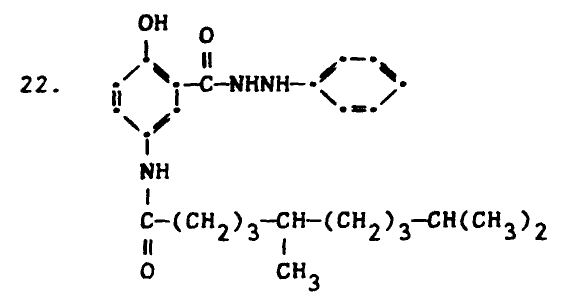

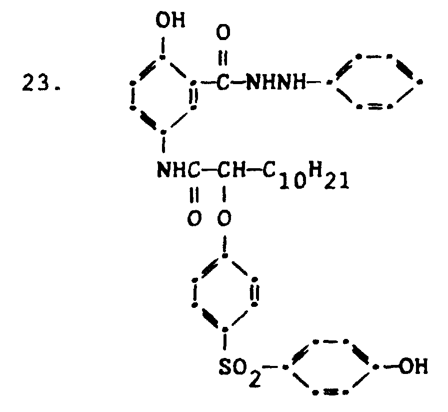

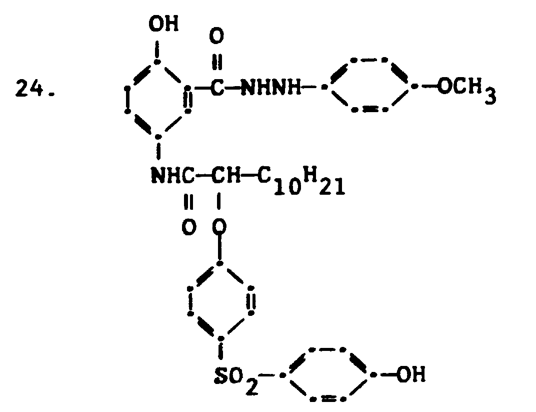

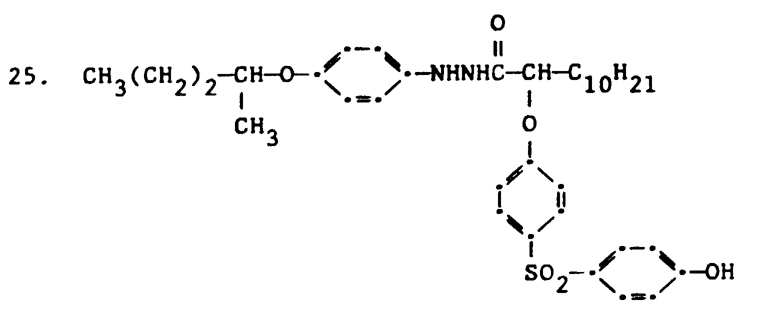

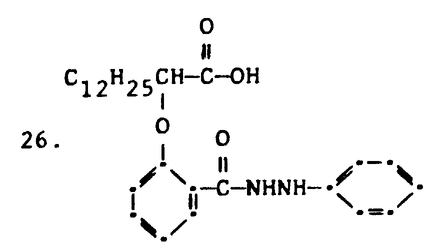

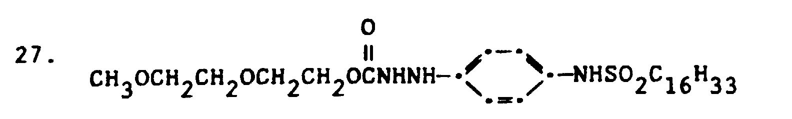

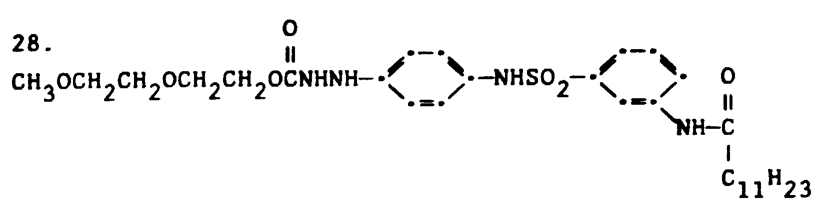

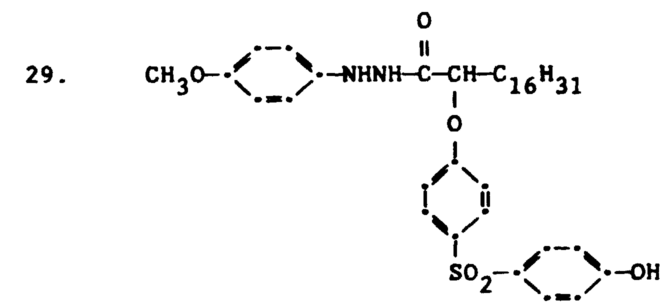

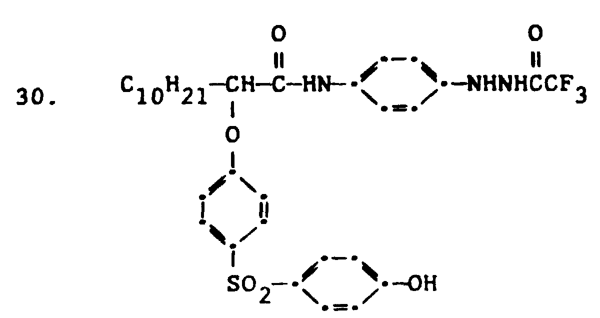

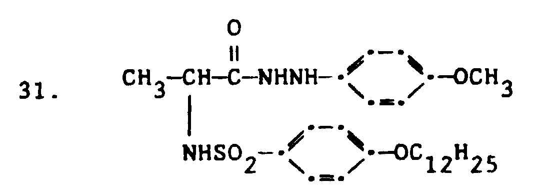

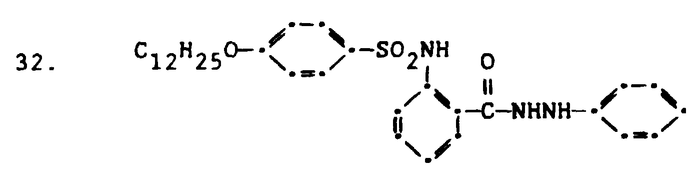

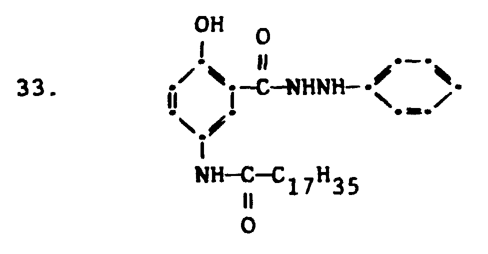

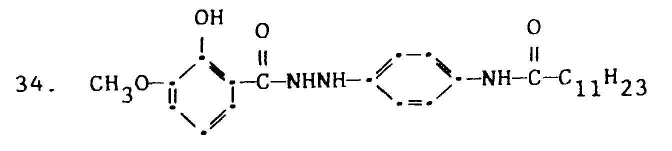

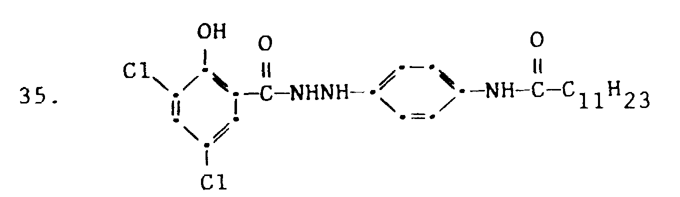

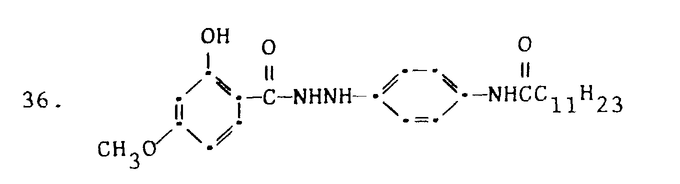

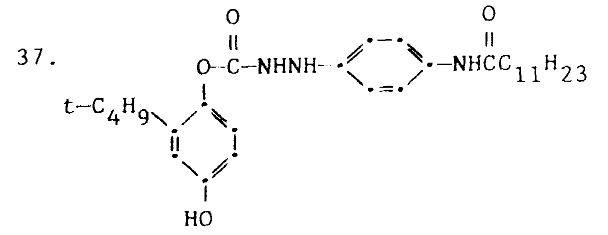

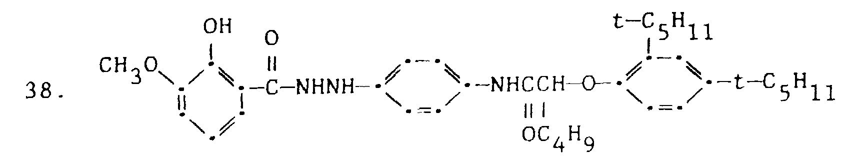

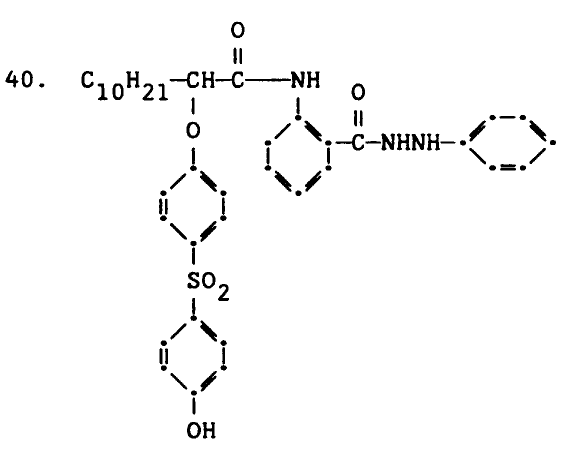

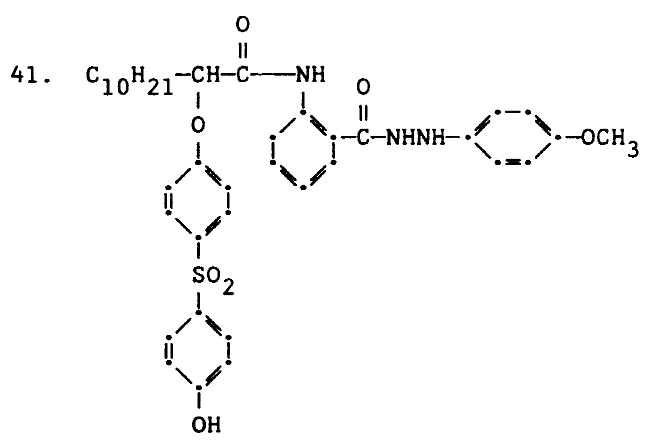

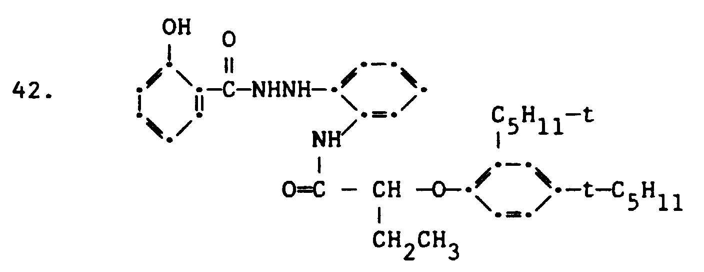

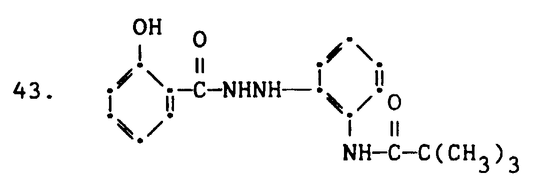

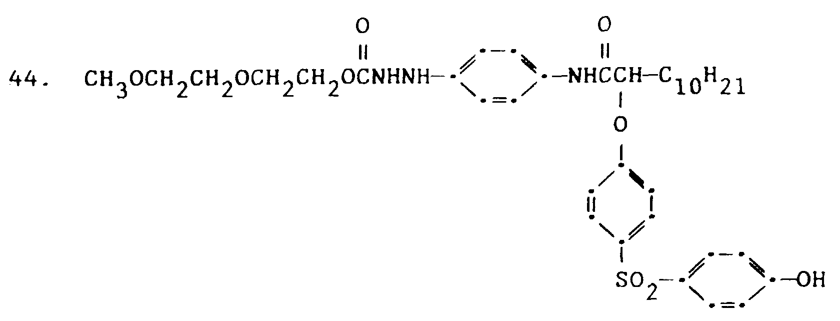

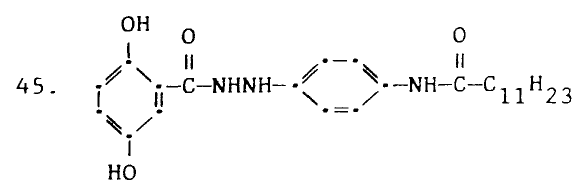

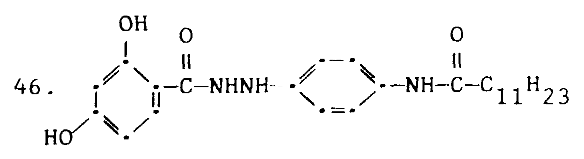

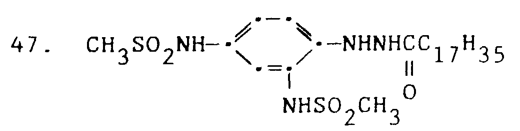

The compounds of the present invention are either known per se or are structurally similar to known compounds. Their preparation can be accomplished using existing technology. For example, Compound No. 1 is prepared according to the following procedure:

### Synthesis of Compound No. 1

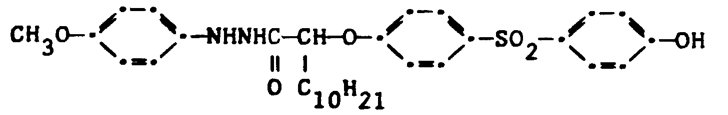

p-methoxyphenylhydrazide hydrochloride (5.0g) was dissolved in acetonitrile (200ml) treated with N,N dimethylaniline (10g) and cooled to < 10°C under nitrogen. The 2-(4-(4-acetoxyphenylsulfonyl)-phenoxy)dodecyl chloride was dissolved in tetrahydrofuran (THF) (50 ml) and added dropwise over a 15 minute period. The reaction mixture was allowed to warm to room temperature while stirring for 2 hours. The reaction was partitioned with ethyl acetate (EtOAc) and 10% HCl. The organic layer was washed again with 10% HCl, dried with MgSO₄ and concentrated to a thick oil.

The thick oil was dissolved in 100 ml of methanol and treated with 5 ml of conc HCl and stirred overnight at room temperature. The solution was partitioned between EtOAC and water and the organic layer was washed with water, separated, dried with MgSO₄ and concentrated to a thick oil. This oil was dissolved in dichloromethane and concentrated (twice) and finally dissolved in 20 ml of dichloromethane and allowed to stand overnight to crystallize. The solid was collected by filtration and washed with cold methylene chloride and air dried to yield 15g (48%) of the desired product (mp 148-150°C).

### Synthesis of Compound No. 21

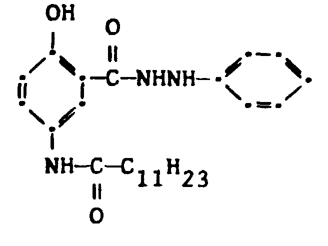

Lauroyl chloride (72.2 g) and pivalic acid (33.7g) were dissolved in 300 ml of acetonitrile and treated with triethylamine (33.4 g). The reaction mixture was stirred at room temperature for 3 hours. 5-aminosalicyclic acid was added to the reaction mixture and the reaction was stirred at room temperature overnight. The solid which formed was filtered off and washed first with acetonitrile, and then with 10% HCl solution. The resulting product was recrystallized from methanol and dried overnight in the vacuum oven at room temperature.

This compound (10g) was treated with 50 ml of thionyl chloride and stirred at room temperature for 3 hours and concentrated to a thick oil. The thick oil was dissolved in 50ml of tetrahydrofuran and added dropwise to a solution of phenylhydrazine (3.2g) and N,N dimethaniline (3.6g) in tetrahydrofuran cooled by a dry ice acetone bath. The resulting reaction mixture was kept cold and stirring for three hours. A solution of aqueous 10% HCl (200ml) was added and the reaction mixture was brought to room temperature. The reaction mixture was partitioned with ethyl acetate. The organic layer was washed with 10% HCl solution (200ml), dried with magnesium sulfate and concentrated. The product was purified by column chromatography on silica gel to yield 9g (70%) of the desired product mp (153°C).

### Synthesis of Compound No. 27

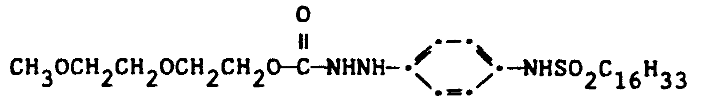

To a round bottom flask containing methoxyethoxyethanol was added phosgene in toluene. This reaction mixture was stirred under nitrogen at room temperature overnight and the reaction was concentrated to one-third volume. This solution was added dropwise to a solution of p-nitrophenylhydrazine hydrochloride (32.2g) and N,N-dimethylaniline (41.2g) in acetonitrile (300ml) and the reaction was stirred at room temperature for 3 hours. The reaction mixture was partitioned with ethyl acetate and 10% HCl solution. The organic layer was washed with 10% HCl solution, dried with magnesium sulfate and then concentrated to a solid. This material (15g) was taken up in tetrahydrofuran (50ml) and treated with 2g of 10% palladium on carbon and shaken with hydrogen at 40 psi overnight. The reaction was treated with hexadecylsulfonylchloride (16.2g) and triethylamine (5.1g) and stirred at room temperature for 3 hours.

The reaction mixture was filtered through super cell, and then partitioned with ethyl acetate and 10% HCl. The organic layer was washed with 10% HCl, dried with magnesium sulfate and concentrated down.

The product was purified with column chromatography using a 30% ethyl acetate/70% dichloromethane solvent system to yield 19g (68%) of the desired product (mp 82°C).

The scavengers of this invention can be used in the ways and for the purposes that scavengers for oxidized developing agent are employed in the art. For example, they can be incorporated in an interlayer between silver halide emulsion layers sensitive to different regions of the visible spectrum. Alternatively, they can be in a layer comprising a color forming material or in a layer comprising other photographic addenda, such as for example, a layer containing a filter dye or a reflecting agent or a mordant. When incorporated in a separate layer, such layer can also be an undercoat layer positioned between all of the silver halide emulsion layers and a support. The separate layer can also be an overcoat layer positioned above all of the silver halide emulsion layers.

The amount of scavenger compound employed will depend upon the particular purpose for which the scavenger is to be used and the degree of scavenging desired. Typically useful results are obtained when the scavenger is employed in an amount of between 5 to 2000 mg/meter².

The scavenger can be incorporated in photographic elements by techniques known in the art. In certain embodiments, the scavenger can be dissolved in a high boiling solvent, such as a water insoluble coupler solvent, and then dispersed either in a silver halide emulsion layer or in a separate vehicle such as gelatin. Typical useful coupler solvents are moderately polar solvents such as tritolylphosphate, di-n-butylphthalate, diethyllauramide and 2,4-dipentylphenol. Typical vehicles are gelatin and other hydrophilic colloids commonly employed in silver halide photographic elements. These vehicles are described in RD, December 1978, Item No. 17643, Section IX. The scavengers can be introduced into the element in a polymeric latex. Suitable techniques for dispersing the scavengers in a latex are described in U. S. Patent Nos. 4,203,716 and 4,214,047 and in RD, July 1977, Item 15930 and July 1980, Item 19551.

Multicolor photographic elements employed in this invention contain dye image forming units sensitive to each of the three primary regions of the spectrum. Each unit can be comprised of a single emulsion layer or of multiple emulsion layers sensitive to a given region of the spectrum. The layers of the element, including the layers of the image-forming units, can be arranged in various order as is known in the art. In an alternative format, the emulsion or emulsion layers can be disposed as one or more segmented layers, e.g., as by the use of microvessels or microcells, as described in U. S. Patent No. 4,362,806.

A preferred photographic element according to this invention comprises a support bearing at least one blue-sensitive silver halide emulsion layer having associated therewith a yellow image dye-providing material, at least one green-sensitive silver halide emulsion layer having associated therewith a magenta image dye-providing material and at least one red-sensitive silver halide emulsion layer having associated therewith a cyan image dye-providing material, the element also containing a scavenger of this invention.

The elements of the present invention can contain additional layers conventional in photographic elements, such as overcoat layers, spacer layers, filter layers, antihalation layers, pH lowering layers (sometimes referred to as acid layers), neutralizing layers, timing layers, opaque reflecting layers and opaque light-absorbing layers. The support can be of any material known to be suitable for use with photographic elements. Typical supports include polymeric films, paper (including polymer-coated paper), metal or glass. Details regarding supports and other layers of the photographic elements useful in this this invention are contained in RD, December 1978, Item 17643, referred to above.

The light-sensitive silver halide emulsions employed in the photographic elements of this invention can include coarse, regular or fine grain silver halide crystals or mixtures thereof and can be comprised of such silver halides as silver chloride, silver bromide, silver bromoiodide, silver chlorobromide, silver chloroiodide, silver chlorobromoiodide, and mixtures thereof. The emulsions can be, for example, tabular grain light-sensitive silver halide emulsions. The emulsions can be negative working or direct positive emulsions. They can form latent images predominantly on the surface of the silver halide grains or in the interior of the silver halide grains. They can be chemically and spectrally sensitized in accordance with usual practices. The emulsions typically will be gelatin emulsions although other hydrophilic colloids can be used in accordance with usual practice. Details regarding the silver halide emulsions are contained in RD, Item 17643, December, 1978 and the references listed therein.

The photographic silver halide emulsions can contain other addenda conventional in the photographic art. Useful addenda are described, for example, in RD, December 1978, Item 17643. Useful addenda include spectral sensitizing dyes and desensitizers, antifoggants, masking couplers, DIR couplers, DIR compounds, anti-stain agents, image dye stabilizers, absorbing materials such as filter dyes and UV absorbers, light scattering materials, coating aids, plasticizers and lubricants, and the like.

Depending upon the dye image-providing material employed in the photographic element, such addenda can be incorporated in the silver halide emulsion layer or in a separate layer associated with the emulsion layer. The dye image-providing material can be any of a number known in the art, such as dye-forming couplers, bleachable dyes, dye developers and redox dye-releasers. The particular material employed will depend on the nature of the element and the type of image desired.

Dye image-providing materials employed with conventional color materials designed for processing with separate solutions are preferably dye-forming couplers; i.e., compounds which couple with oxidized developing agent to form a dye. Preferred couplers which form cyan dye images are phenols and napthols. Preferred couplers which form magenta dye images are pyrazolones and pyrazolotriazoles. Preferred couplers which form yellow dye images are benzoylacetanilides and pivalylacetanilides.

The developing agents which can be used to develop the photographic elements of this invention, the oxidized form of which can be reduced by the scavengers of this invention, include hydroquinones, aminophenols, 3-pyrazolidones and phenylenediamines. Some of these developing agents, when used for certain applications, are referred to in the art as electron transfer agents. The particular developing agent employed will depend on the particular type of photographic element to be processed. For example, phenylenediamines are the developers of choice for use with color photographic elements containing dye-forming couplers, while 3-pyrazolidones are frequently used with image transfer materials containing redox dye releasers.

Representative developing agents include: hydroquinone, N-methylaminophenol; 1-phenyl-3-pyrazolidone; 1-phenyl-4,4-dimethyl-3-pyrazolidone; 1-phenyl-4-methyl-4-hydroxymethyl-3-pyrazolidone; N,N-diethyl-p-phenylenediamine; 3-methyl-N,N-diethyl-p-phenylenediamine; 3-methoxy-N,N-diethyl-p-phenylenediamine; and N,N,N',N'-tetramethyl-p-phenylenediamine.

The term "non-diffusible" used herein has the meaning commonly applied to the term in photography and denotes materials that for all practical purposes do not migrate or wander through organic colloid layers of a photographic element, such as gelatin, when the element is processed in an alkaline medium, preferably when processed in a medium having a pH of 10 or greater. The term "diffusible" has the converse meaning and denotes the materials having the property of diffusing effectively through the colloid layers of photographic elements in an alkaline medium.

The term "associated therewith" as used herein is intended to mean that the materials can be in either the same or different layers so long as the materials are accessible to one another during processing.

The following examples further illustrate this invention.

### Example 1

Compounds of the invention were evaluated in a two layer format (imaging layer and overcoat). The imaging layer was coated on a cellulose acetate support and consisted of 1.5 g/m² gelatin, 1.1 g/m² of image Coupler A, 1.75 g per Ag mole of tetraazaindene, 0.75 g/m² of silver halide emulsion and a compound of this invention in the amount indicated in Table I. Each emulsion was comprised of silver bromoiodide grains (2% iodide; 0.53 micron) which were green sensitized with Dyes B and C noted below. The overcoat consisted of 2.0 g/m² of gelatin. The coatings were hardened with bis(vinylsulfonylmethyl)ether at a level of 1.75% of the total gelatin. Saponin was used as a surfactant in both layers. The compounds were dispersed at a 1:2 ratio in N,N-diethyldecanamide using cyclohexanone (1:3 ratio) as auxillary solvent. The dispersions were washed for 6 hours prior to coating.

Portions of the coatings were placed in a bomb containing air at 4000 psi (27,580 Pa) and 50°C, and held for 6, 40, and 72 hours. The strips were then extracted to determine the amount of compound still remaining. (The bomb test is an accelerated test which is indicative of long term resistance of the compound being tested to oxidation).

Stability results were as follows:

**Table I**

| Invention Compound No. | | Percent Compound Remaining | | |
|---|---|---|---|---|
| | mg/m² | 6 hours | 40 hours | 72 hours |
| 1 | 240 | 100% | 93% | 87% |
| 2 | 76 | 95% | 84% | 79% |

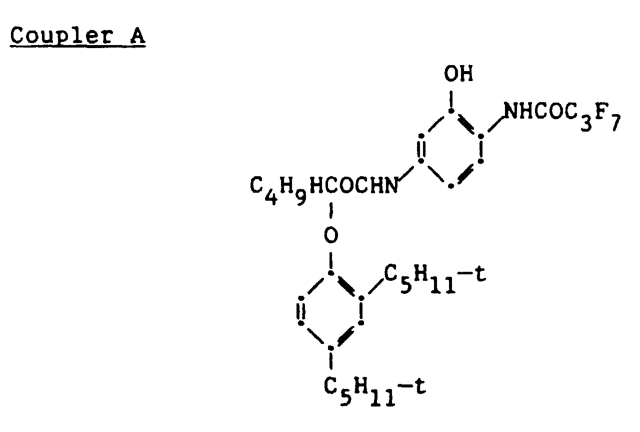

### Sensitizing Dye B

### Anhydro-5,5',6,6'-tetrachloro-1,1',3-triethyl-3'-(3-sulfobutyl)-benzimidazolocarbocyanine hydroxide

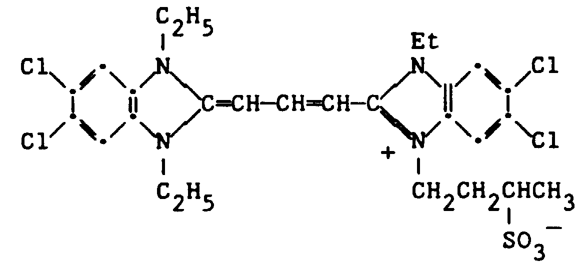

### Sensitizing Dye C

### Anhydro-5,5'-dichloro-9-ethyl-3,3'-di(3-sulfopropyl)-oxacarbocyanine hydroxide, sodium salt

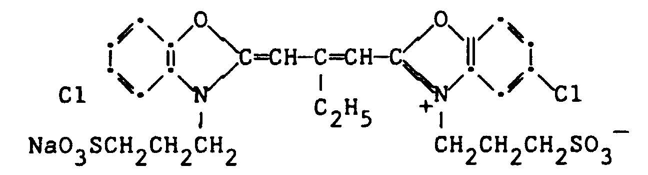

From the above data it can be seen that compounds of this invention are stable under the conditions of the test and that decomposition is avoided. Decomposition results in stain formation in the imaging layers.

### Example 2

Additional tests were run using coatings prepared as described in Example 1. The purpose of these tests was to show the effects of compounds of this invention on dye density values.

Each example was exposed using a 21 step tablet ranging from 0 to 3.0 density in steps of 0.15 with a 5500K illuminant for 0.02 sec. through a Wratten 8 filter. Each sample was processed in an E-6 processing sequence.

The compounds tested were coated at two levels, 0.17 mmol/m² and 0.42 mmol/m², which levels correspond to 10 and 25 mol % of the amount of coupler coated. Results from the higher coating level are reported in Table II.

**Table II**

| Compound No. | Toe Density (Step 7) | | Density (Step 15) | | Stain | |
|---|---|---|---|---|---|---|
| | | | | | Blue | Green |
| 13 | .08 | .17 | .41 | .61 | .04 | .03 |
| 3 | .08 | .14 | .19 | .42 | .04 | .03 |
| 1 | .19 | .34 | .23 | .64 | .04 | .02 |
| 21 | .17 | .27 | .17 | .40 | .04 | .03 |
| 22 | .18 | .24 | .24 | .46 | .04 | .03 |
| 23 | .14 | .17 | .21 | .33 | .03 | .02 |
| 24 | .14 | .20 | .24 | .41 | .04 | .04 |
| 25 | .15 | .21 | .32 | .66 | .04 | .03 |
| 27 | .12 | .13 | .14 | .43 | .04 | .03 |
| 38 | .20 | .28 | .44 | .80 | .04 | .03 |

As can be seen from the above data compounds if this invention, even when coated at 0.42 mmol/m², cause minimal blue or green stain.

## Claims

1. A color photographic element comprising a support, at least one silver halide emulsion layer and a scavenger compound for oxidized developing agent characterized in that the scavenger compound is a hydrazide having the structural formula: 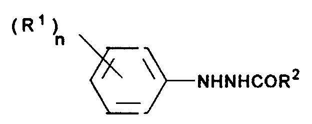 wherein:
R¹ is alkyl of 1 to 20 carbon atoms, alkoxy of 1 to 20 carbon atoms, carboxy, carbonamido having the formula -NR⁴COR⁵, -OCOR⁵, sulphonamido having the formula -NR⁴SO₂R⁵, or amino having the formula -NR⁴R⁵, wherein R⁴ is hydrogen or alkyl of 1 to 8 carbon atoms, and R⁵ is hydrogen, alkyl of 1 to 16 carbon atoms, a benzyl group or a phenyl group;
R² represents hydrogen, alkyl of 1 to 20 carbon atoms, alkoxy of 1 to 20 carbon atoms, aryl having 6 to 10 ring carbon atoms, aryloxy having 6 to 10 ring carbons, aralkyl or amino of the formula -NHR³ wherein R³ is phenyl or benzyl, and
n is 0, 1 or 2,
provided that at least one of the substituents R¹ and R² represents a ballast group of sufficient size as to render the hydrazide compound non-diffusible in the photographic element prior to development in alkaline processing solution,
further provided that at least one of the substituents R¹ and R² comprises a polar group which is -NHSO₂CH₃, -NHSO₂C₁₆H₃₃, -NHSO₂aryl, -CH₂OH, -NH₂, -COOH, -CONH₂, -NHCONH₂, -NHCSNH₂, -N⁺(R⁵)₃, -SO₃⁻, -SO₂⁻, 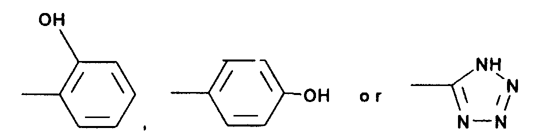 and still further provided that the hydrazide compound does not contain a timing group which releases a photographically useful group.

2. The photographic element of claim 1 characterized in that at least one R¹ substituent is an alkyl or alkoxy group having from 8 to 20 carbon atoms, or a carboxy, carbonamido, sulfonamido or amino group as defined.

3. The photographic element of claim 2 characterized in that R¹ is alkyl or alkoxy having from 8 to 16 carbon atoms.

4. The photographic element of any of claims 1 - 3 characterized in that R² is alkyl or alkoxy having from 8 to 20 carbon atoms or an aryl or aryloxy having from 6 to 10 ring carbon atoms.

5. The photographic element of any of claims 1 - 4 characterized in that R² is phenyl or phenoxy optionally substituted with a hydrogen bonding group in a position ortho to the point of attachment of the carbonyl group.

6. The photographic element of claim 5 characterized in that the hydrogen bonding group is hydroxy, primary or secondary amino of the formula -NR⁴R⁵, sulfonamido of the formula -NHSO₂R⁴, carbonamido of the formula -NHCOR⁴ or ureido of the formula -NHCONHR⁴.

7. The photographic element of any of claims 1 - 6 characterized in that the scavenger compound is present in a layer located between silver halide emulsion layers sensitive to different regions of the visible spectrum, in a layer comprising a color-forming agent or other photographic addenda, or in an overcoat or undercoat layer.

8. The photographic element of any of claims 1 - 7 characterized in that the scavenger compound is present in an amount of from 5 to 2000 mg/m².

9. The photographic element of any of claims 1 - 8 wherein the ballast group in the scavenger compound renders that compound non-diffusible in the photographic element prior to development in an alkaline processing medium having a pH of 10 or greater.

10. The photographic element of claim 1, 7 or 8 characterized in that the scavenger compound has one of the following structural formulae: 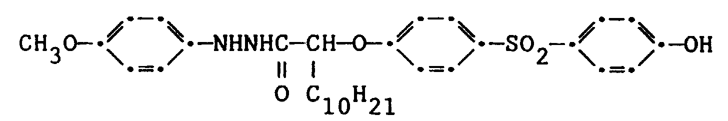 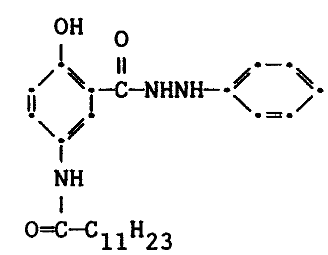 or

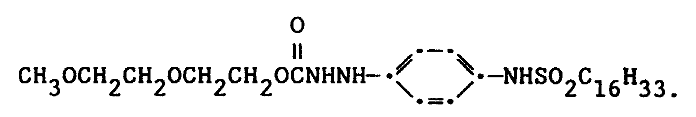

## Patentansprüche

1. Farbphotographisches Element mit einem Träger, mindestens einer Silberhalogenidemulsionsschicht und einer Abfang-Verbindung für oxidierte Entwicklerverbindung, dadurch gekennzeichnet, daß die Abfang-Verbindung ein Hydrazid mit der Strukturformel ist: 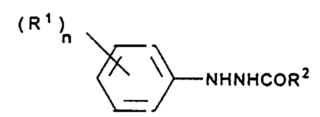 worin:
R¹ steht für Alkyl mit 1 bis 20 Kohlenstoffatomen, Alkoxy mit 1 bis 20 Kohlenstoffatomen, Carboxy, Carbonamido mit der Formel -NR⁴COR⁵, -OCOR⁵, Sulphonamido mit der Formel -NR⁴SO₂R⁵ oder Amino mit der Formel -NR⁴R⁵, worin R⁴ steht für Wasserstoff oder Alkyl mit 1 bis 8 Kohlenstoffatomen und worin R⁵ steht für Wasserstoff, Alkyl mit 1 bis 16 Kohlenstoffatomen, eine Benzylgruppe oder eine Phenylgruppe;
R² steht für Wasserstoff, Alkyl mit 1 bis 20 Kohlenstoffatomen, Alkoxy mit 1 bis 20 Kohlenstoffatomen, Aryl mit 6 bis 10 Ringkohlenstoffatomen, Aryloxy mit 6 bis 10 Ringkohlenstoffatomen, Aralkyl oder Amino mit der Formel -NHR³, worin R³ steht für Phenyl oder Benzyl, und
n steht für 0, 1 oder 2,
wobei gilt, daß mindestens einer der Substituenten R¹ und R² eine Ballastgruppe einer ausreichenden Größe darstellt, um die Hydrazid-Verbindung in dem photographischen Element vor der Entwicklung in alkalischer Entwicklungslösung nicht-diffundierend zu machen,
wobei ferner gilt, daß mindestens einer der Substituenten R¹ und R² eine polare Gruppe aufweist, die besteht aus -NHSO₂CH₃, -NHSO₂C₁₆H₃₃, -NHSO₂aryl, -CH₂OH, -NH₂, -COOH, -CONH₂, -NHCONH₂, -NHCSNH₂, -N⁴(R⁵)₃, -SO₃⁻, -SO₂⁻, 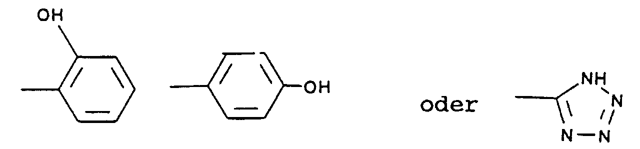 und wobei ferner gilt, daß die Hydrazid-Verbindung keine Zeitsteuergruppe aufweist, die eine photographisch geeignete Gruppe freigibt.

2. Photographisches Element nach Anspruch 1, dadurch gekennzeichnet, daß mindestens ein R¹-Substituent eine Alkyl- oder Alkoxygruppe mit 8 bis 20 Kohlenstoffatomen ist oder eine Carboxy-, Carbonamido-, Sulfonamido- oder Aminogruppe wie definiert.

3. Photographisches Element nach Anspruch 2, dadurch gekennzeichnet, daß R¹ steht für eine Alkyl- oder Alkoxygruppe mit 8 bis 16 Kohlenstoffatomen.

4. Photographisches Element nach einem der Ansprüche 1 - 3, dadurch gekennzeichnet, daß R² steht für eine Alkyl- oder Alkoxygruppe mit 8 bis 20 Kohlenstoffatomen oder eine Aryl- oder Aryloxygruppe mit 6 bis 10 Ringkohlenstoffatomen.

5. Photographisches Element nach einem der Ansprüche 1 - 4, dadurch gekennzeichnet, daß R² steht für eine Phenyl- oder Phenoxygruppe, die gegebenenfalls substituiert ist durch eine Wasserstoff bindende Gruppe in einer Position Ortho zum Punkt der Bindung der Carbonylgruppe.

6. Photographisches Element nach Anspruch 5, dadurch gekennzeichnet, daß die Wasserstoff bindende Gruppe besteht aus einer Hydroxygruppe, einer primären oder sekundären Aminogruppe der Formel -NR⁴R⁵, einer Sulfonamidogruppe der Formel -NHSO₂R⁴, einer Carbonamidogruppe derFormel -NHCOR⁴ oder einer Ureidogruppe der Formel -NHCONHR⁴.

7. Photographisches Element nach einem der Ansprüche 1 - 6, dadurch gekennzeichnet, daß die Abfang-Verbindung in einer Schicht vorliegt, die angeordnet ist zwischen Silberhalogenidemulsionsschichten, die gegenüber unterschiedlichen Bereichen des sichtbaren Spektrums empfindlich sind, in einer Schicht mit einem Farbe erzeugenden Mittel oder anderen photographischen Zusätzen, oder in einer Überzugs- oder Unterschicht.

8. Photographisches Element nach einem der Ansprüche 1 - 7, dadurch gekennzeichnet, daß die Abfang-Verbindung in einer Menge von 5 bis 2000 mg/m² vorliegt.

9. Photographisches Element nach einem der Ansprüche 1 - 8, in dem die Ballastgruppe in der Abfang-Verbindung die Verbindung nicht-diffundierend in dem photographischen Element vor der Entwicklung in einem alkalischen Entwicklungsmedium mit einem pH-Wert von 10 oder größer macht.

10. Photographisches Element nach Anspruch 1, 7 oder 8, dadurch gekennzeichnet, daß die Abfang-Verbindung eine der folgenden Strukturformeln aufweist: 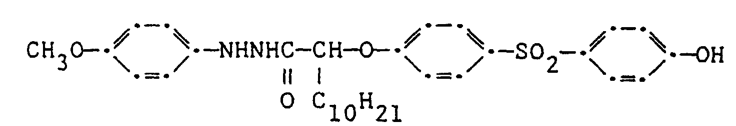 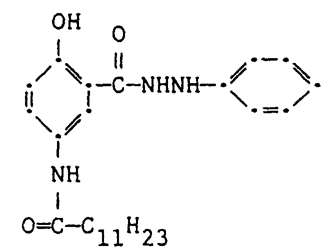 oder

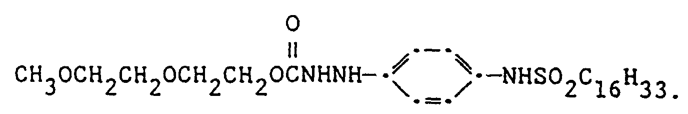

## Revendications

1. Elément photographique couleur comprenant un support, au moins une couche d'émulsion aux halogénures d'argent et un agent d'immobilisation pour le développateur oxydé, caractérisé en ce que l'agent d'immobilisation est un hydrazide ayant la formule développée : 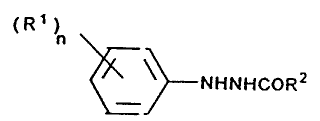 où :
R¹ est un groupe alkyle ayant 1 à 20 atomes de carbone, un groupe alkoxy ayant 1 à 20 atomes de carbone, un groupe carboxy, un groupe carbonamido ayant la formule -NR⁴COR⁵, -OCOR⁵, un groupe sulfonamido ayant la formule -NR⁴SO₂R⁵, ou un groupe amino ayant la formule -NR⁴R⁵, dans lesquelles R⁴ est un hydrogène ou un groupe alkyle ayant 1 à 8 atomes de carbone, et R⁵ est un hydrogène, un groupe alkyle ayant 1 à 16 atomes de carbone, un groupe benzyle ou un groupe phényle ;
R² représente un hydrogène, un groupe alkyle ayant 1 à 20 atomes de carbone, un groupe alkoxy ayant 1 à 20 atomes de carbone, un groupe aryle ayant 6 à 10 atomes de carbone nucléaire, un groupe aryloxy ayant 6 à 10 atomes de carbone nucléaire, un groupe aralkyle ou un groupe amino ayant la formule -NHR³, dans laquelle R³ est un groupe phényle ou benzyle, et
n est égal à 0, 1 ou 2,
à condition qu'au moins l'un des substituants R¹ et R² représente un groupe ballast de taille suffisante pour rendre le composé hydrazide non diffusible dans l'élément photographique avant le développement dans une solution de traitement alcaline,
à condition aussi qu'au moins l'un des substituants R¹ et R² comprenne un groupe polaire qui est -NHSO₂CH₃, -NHSO₂C₁₆H₃₃, -NHSO₂aryle, -CH₂OH, -NH₂, -COOH, -CONH₂, -NHCONH₂, -NHCSNH₂, -N⁺(R⁵)₃, -SO₃⁻, -SO₂⁻, 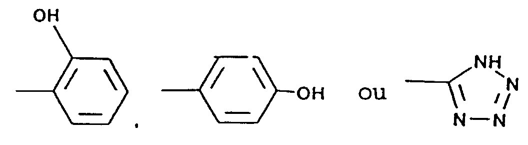 et à condition aussi que le composé hydrazide ne contienne pas de groupe retardateur qui libère un groupe photographiquement utile.

2. Elément photographique selon la revendication 1, caractérisé en ce qu'au moins l'un des substituants R¹ est un groupe alkyle ou alkoxy ayant 8 à 20 atomes de carbone, ou un groupe carboxy, carbonamido, sulfonamido ou amino, tels que définis.

3. Elément photographique selon la revendication 2, caractérisé en ce que R¹ est un groupe alkyle ou alkoxy ayant 8 à 16 atomes de carbone.

4. Elément photographique selon l'une quelconque des revendications 1 à 3, caractérisé en ce que R² est un groupe alkyle ou alkoxy ayant 8 à 20 atomes de carbone ou un groupe aryle ou aryloxy ayant 6 à 10 atomes de carbone nucléaire.

5. Elément photographique selon l'une quelconque des revendications 1 à 4, caractérisé en ce que R² est un groupe phényle ou phénoxy éventuellement substitué par un groupe en position ortho par rapport au point de rattachement du groupe carbonyle fournissant une liaison hydrogène.

6. Elément photographique selon la revendication 5, caractérisé en ce que le groupe fournissant une liaison hydrogène est un groupe hydroxy, un groupe amino primaire ou secondaire de formule -NR⁴R⁵, un groupe sulfonamido de formule -NHSO₂R⁴, un groupe carbonamido de formule -NHCOR⁴ ou un groupe uréido de formule -NHCONHR⁴.

7. Elément photographique selon l'une quelconque des revendications 1 à 6, caractérisé en ce que l'agent d'immobilisation se trouve dans une couche située entre des couches d'émulsion aux halogénures d'argent sensibles à différentes régions du spectre visible, dans une couche comprenant un agent chromogène ou d'autres additifs photographiques, dans une surcouche ou dans une sous-couche.

8. Elément photographique selon l'une quelconque des revendications 1 à 7, caractérisé en ce que l'agent d'immobilisation est présent en une quantité allant de 5 à 2000 mg/m².

9. Elément photographique selon l'une quelconque des revendications 1 à 8, dans lequel le groupe ballast contenu dans l'agent d'immobilisation rend ce composé non diffusible dans l'élément photographique avant le développement dans un milieu de traitement alcalin ayant un pH supérieur ou égal à 10.

10. Elément photographique selon la revendication 1, 7 ou 8, caractérisé en ce que l'agent d'immobilisation a l'une des formules développées suivantes : 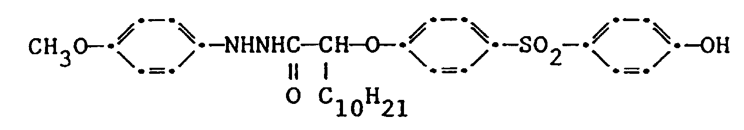 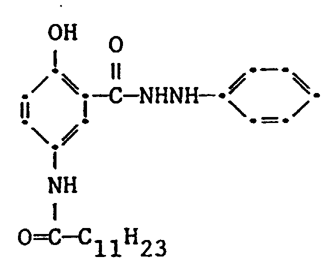 ou
